# EUROPEAN PATENT SPECIFICATION

(11) **EP 4 385 432 B1**
(45) Date of publication and mention of the grant of the patent: **25.11.2026**
(21) Application number: 23215498.9
(22) Date of filing: 11.12.2023
(51) Int. Cl.: A61B 17/42, A61B 34/20, A61B 17/32, A61B 90/00, A61B 90/30, A61B 17/34

(54) **SYSTEMS FOR TRACKING SURGICAL INSTRUMENTS**
SYSTEME ZUR VERFOLGUNG VON CHIRURGISCHEN INSTRUMENTEN
SYSTÈMES DE SUIVI D'INSTRUMENTS CHIRURGICAUX

(30) Priority: 13.12.2022 US 202218080098
(43) Date of publication of application: 19.06.2024
(73) Proprietor: Covidien LP, Mansfield, MA 02048 (US)
(72) Inventor: MONTAYRE, Rommel N., Statham, 30666 (US); PRIOR, Scott J., North Haven, 06473 (US); WHISLER, Jordan A., Woburn, 01801 (US); BEGG, Nikolai, Woburn, 01801 (US)
(74) Representative: Wallin, Nicholas James

(56) References cited:
- US-A1- 2013 261 392
- US-A1- 2015 208 947
- US-A1- 2015 265 367
- US-A1- 2021 251 695

## Description

### FIELD

The present disclosure relates to systems for tracking of surgical instruments and, more specifically, to systems for linear and rotational tracking of surgical instruments.

### BACKGROUND

Tracking surgical instruments during surgical procedures facilitates performing the surgical procedures and increases patient safety. For example, with respect to transvaginal hysteroscopic surgical procedures, instrument tracking facilitates positioning an instrument at particular anatomical location (e.g., within the vagina, vaginal fornix, cervix, uterus, uterine wall, or in any other suitable for location) and relative to tissue of interest to operate on (e.g., cut, ablate, observe, biopsy, remove, join, treat (thermally, electrically, mechanically, and/or chemically), etc.), the tissue of interest while avoiding damage to surrounding tissue. US-2021/251695A1 describes an endoscopy system.
US-2015/208947A1 describes an insertion system having an insertion portion and insertion member. US-2013/261392A1 describes a tubular insertion system.
US-2015/265367A1 describes automatic registration of the penetration depth and the rotational orientation of an invasive instrument.

### SUMMARY

The present invention is defined by appended claim 1. Specific embodiments are set forth in the dependent claims. As used herein, the term "distal" refers to the portion that is being described which is farther from an operator (whether a human surgeon or a surgical robot), while the term "proximal" refers to the portion that is being described which is closer to the operator. Terms including "generally," "about," "substantially," and the like, as utilized herein, are meant to encompass variations, e.g., manufacturing tolerances, material tolerances, use and environmental tolerances, measurement variations, design variations, and/or other variations, up to and including plus or minus 10 percent. Further, to the extent consistent, any of the aspects described herein may be used in conjunction with any or all of the other aspects described herein.

Provided in accordance with examples of the present disclosure is a system for tracking a surgical instrument that includes a pattern and a sensor assembly. The pattern is on a first surgical instrument and has a plurality of first markers longitudinally spaced relative to one another and a plurality of second markers circumferentially spaced relative to one another. The sensor assembly is configured to operably coupled to a second surgical instrument and includes at least one sensor. The at least one sensor is configured to detect the at least one first marker of the plurality of first markers, thereby enabling determination of longitudinal translation of the first surgical instrument relative to the second surgical instrument and to detect the intersection of at least one second marker of the plurality of second markers, thereby enabling determination of rotation of the first surgical instrument relative to the second surgical instrument.

In an example of the present disclosure, the at least one sensor includes at least one first sensor configured to detect the at least one first marker of the plurality of first markers, and at least one second sensor configured to detect the at least one second marker of the plurality of second markers.

In another example of the present disclosure, the at least one sensor includes at least two first sensors. Each sensor of the at least two first sensors is configured to detect the at least one first marker of the plurality of first markers. The at least two first sensors are longitudinally spaced relative to one another.

In still another example of the present disclosure, the at least two first sensors enable determination of a direction of the longitudinal translation of the first surgical instrument relative to the second surgical instrument.

In yet another example of the present disclosure, the at least one sensor includes at least two second sensors. Each of the at least two second sensors is configured to detect the at least one second marker of the plurality of second markers. The at least two second sensors are circumferentially spaced relative to one another.

In still yet another example of the present disclosure, the at least two second sensors enable determination of a direction of the rotation of the first surgical instrument relative to the second surgical instrument.

In another example of the present disclosure, the at least one sensor is an optical sensor and the plurality of first markers is optically distinct from the plurality of second markers.

A surgical system for surgical instrument tracking provided in accordance with the present disclosure includes a first surgical instrument, a second surgical instrument, and a sensor assembly. The first surgical instrument includes a pattern having a plurality of first markers arranged for detection in response to longitudinal translation of the first surgical instrument, and a plurality of second markers arranged for detection in response to rotation of the first surgical instrument. The second surgical instrument is configured to guide the first surgical instrument such that the first surgical instrument is permitted to translate longitudinally and rotate relative to the second surgical instrument and such that the first surgical instrument is constrained relative to the second surgical instrument in at least one other degree of freedom. The sensor assembly is operably coupled to the second surgical instrument and includes at least one sensor defining a sensing region. The at least one sensor is configured to detect at least one first marker of the plurality of first markers, thereby enabling determination of longitudinal translation of the first surgical instrument relative to the second surgical instrument, and to detect at least one second marker of the plurality of second markers, thereby enabling determination of rotation of the first surgical instrument relative to the second surgical instrument.

In an aspect of the present disclosure, the second surgical instrument defines a passage configured to receive at least a portion of the first surgical instrument, and the first surgical instrument is configured to longitudinally translate and rotate within and relative to the passage of the second surgical instrument.

In another aspect of the present disclosure, a sensing region of the at least one sensor intersects the passage or an extension volume of the passage.

In still another aspect of the present disclosure, the pattern is disposed on an exterior cylindrical surface of the first surgical instrument.

In another aspect of the present disclosure, the sensor assembly is integrated into the second surgical instrument. Alternatively, the sensor assembly may be removably coupled to the second surgical instrument.

In yet another aspect of the present disclosure, the at least one sensor includes at least one first sensor configured to detect the at least one first marker of the plurality of first markers, and at least one second sensor configured to detect the at least one second marker of the plurality of second markers.

In still yet another aspect of the present disclosure, the at least one sensor includes at least two first sensors each configured to detect the at least one first marker of the plurality of first markers, and at least two second sensors each configured to detect the at least one second marker of the plurality of second markers.

In another aspect of the present disclosure, the at least two first sensors enable determination of a direction of the longitudinal translation of the first surgical instrument relative to the second surgical instrument and/or the at least two second sensors enable determination of a direction of the rotation of the first surgical instrument relative to the second surgical instrument.

In another aspect of the present disclosure, the at least one sensor is an optical sensor and the plurality of first markers is optically distinct from the plurality of second markers.

A method of tracking a surgical instrument in accordance with an example of the present disclosure includes: determining longitudinal translation of a first surgical instrument relative to a second surgical instrument by detecting at least one first marker associated with the first surgical instrument with at least one first sensor associated with the second surgical instrument; and determining rotation of the first surgical instrument relative to the second surgical instrument by detecting at least one second marker associated with the first surgical instrument with at least one second sensor associated with the second surgical instrument.

In an example of the present disclosure, determining the longitudinal translation of the first surgical instrument relative to the second surgical instrument includes determining longitudinal translation of the first surgical instrument through a passage defined by the second surgical instrument, and determining the rotation of the first surgical instrument relative to the second surgical instrument includes determining rotation of the first surgical instrument within the passage defined by the second surgical instrument.

In another example of the present disclosure, the method further includes determining a direction of the longitudinal translation of the first surgical instrument relative to the second surgical instrument and/or determining a direction of the rotation of the first surgical instrument relative to the second surgical instrument.

### BRIEF DESCRIPTION OF DRAWINGS

The above and other aspects and features of the present disclosure will become more apparent in view of the following detailed description when taken in conjunction with the accompanying drawings wherein like reference numerals identify similar or identical elements.
FIG. 1 is an exploded, perspective view of a surgical system in accordance with the present disclosure;
FIG. 2 is a side, partial cross-sectional view of the system of FIG. 1 positioned intravaginally for performing a procedure on or within a uterus;
FIG. 3 is a perspective view of another surgical system in accordance with the present disclosure;
FIG. 4A is a perspective view of a body of a guide instrument in accordance with the present disclosure;
FIG. 4B is a perspective view of a guide piece of the guide instrument of FIG. 4A;
FIG. 4C is a side view of the guide instrument of FIG. 4A including the guide piece assembled on the body;
FIG. 5 is a perspective view of a tracking system in accordance with the present disclosure configured to track a first surgical instrument relative to a second surgical instrument;
FIGS. 6A and 6B illustrate the tracking system of FIG. 5 tracking longitudinal translation of the first surgical instrument relative to the second surgical instrument;
FIGS. 7A and 7B illustrate the tracking system of FIG. 5 tracking rotation of the first surgical instrument relative to the second surgical instrument;
FIGS. 8A-8E are perspective views of other sensor assemblies configured for use with the tracking system of FIG. 5; and
FIG. 9 is a perspective view of another tracking system in accordance with the present disclosure configured to track a first surgical instrument relative to a second surgical instrument.

### DETAILED DESCRIPTION

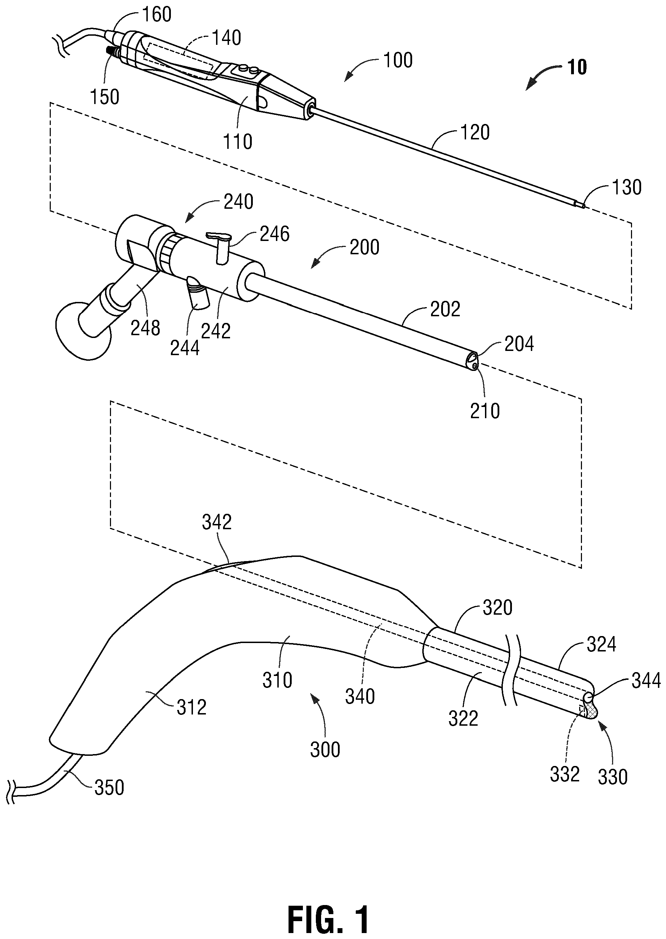

Referring to FIG. 1, a surgical system provided in accordance with the present disclosure is shown generally identified by reference numeral 10. System 10 includes at least two surgical instruments 100, 200, 300 configured to operably couple to one another such that relative movement therebetween is constrained in at least one degree of freedom and such that relative movement therebetween is permitted in at least two degrees of freedom. For example, surgical instrument 100 may be configured for insertion through surgical instrument 200 such that rotation and axial translation of surgical instrument 100 relative to surgical instrument 200 are permitted while transverse motion and tilting of surgical instrument 100 relative to surgical instrument 200 in at least one degree of freedom are substantially constrained. Additionally or alternatively, surgical instrument 200 may be configured for insertion through surgical instrument 300 such that rotation and axial translation of surgical instrument 200 relative to surgical instrument 300 are permitted while transverse motion and tilting of surgical instrument 200 relative to surgical instrument 300 in at least one degree of freedom are substantially constrained.

As detailed below, the present disclosure provides devices and systems for linear and rotational tracking of surgical instruments (e.g., between surgical instruments 100 and 200 and/or between surgical instruments 200 and 300). System 10, as well as the other surgical systems provided herein, are detailed herein as hysteroscopic surgical systems configured for use in hysteroscopic surgical procedures; however, it is understood that the aspects and features of the present disclosure may be utilized in any suitable surgical system. Further, while exemplary instruments 100, 200, 300 are detailed herein, it is likewise understood that the aspects and features of the present disclosure also to apply to any suitable surgical instruments and combinations of surgical instruments including less than or greater than three instruments.

Continuing with reference to FIG. 1, system 10 includes: a working instrument 100 (e.g., a tissue resection device, an ablation device, a biopsy device, a surgical grasper, a surgical stapler, an electrosurgical instrument, etc.); an access instrument 200 (e.g., an endoscope, a cannula, a sheath, etc.) which may also provide visualization capabilities (e.g., in aspects where access instrument 200 is an endoscope or visualization cannula); and, in aspects, a guide instrument 300 which may also provide imaging capabilities (e.g., in aspects where guide instrument 300 is an ultrasound device). With respect to system 10 configured for use in hysteroscopic surgical procedures, as shown in FIG. 1, for example, access instrument 200 may be a hysteroscope (thus also providing visualization capabilities) and/or guide instrument 300 may be an ultrasound device (thus also providing imaging capabilities). Further, for use in hysteroscopic surgical procedures, guide instrument 300 may be configured for trans-vaginal insertion into position adjacent to or in abutment with the cervix, while access instrument 200 is configured for insertion through guide instrument 300 and the cervix into the uterus. Working instrument 100, in such configurations, is configured for insertion through access instrument 200 and into the uterus to perform a surgical procedure in the uterine cavity and/or within the uterine wall.

Working instrument 100 is illustrated in FIG. 1 as a tissue resection device including, for example, a housing 110, a shaft 120, a cutting member 130, a drive mechanism 140, an outflow port 150, and a cable 160. Housing 110 houses drive mechanism 140 therein and functions as a handle to enable a user to grasp working instrument 100, although housing 110 may alternatively mount on a surgical robot arm (not shown). Drive mechanism 140 includes a motor (not shown) and is operably coupled to cutting member 130 to drive rotation and/or translation of cutting member 130 relative to shaft 120. Drive mechanism 140 is adapted to connect to a control unit (not shown) via cable 160 for powering and controlling the motor, although working instrument 100 may alternatively be battery powered or manually powered. A suction source (not shown) incorporated into the control unit, or any other suitable vacuum-creating mechanism, may also be provided to facilitate withdrawal of fluid, tissue, and debris through working instrument 100 and outflow tube 150. Other suitable working instruments are also contemplated such as, for example, an ablation instrument or any other suitable surgical instrument configured to operate on (e.g., cut, ablate, observe, biopsy, remove, join, treat (thermally, electrically, mechanically, and/or chemically), etc.) tissue of interest.

Shaft 120 of working instrument 100 extends distally from housing 110 and, in embodiments, is stationary relative to housing 110, although other configurations are also contemplated. Shaft 120 defines a window through a sidewall thereof towards a distal end of shaft 120 to provide access to cutting member 130 which is rotatably and/or translatably disposed within shaft 120 and, as noted above, operably coupled to drive mechanism 140. Cutting member 130 defines an opening providing access to the interior thereof and may include a serrated cutting edge surrounding the opening, although other suitable cutting edge configurations are also contemplated. Alternatively or additionally, shaft 120 may include a cutting edge defined about the window thereof.

In use of working instrument 100, upon activation, tissue is drawn through the window of shaft 120 and into the opening of cutting member 130, e.g., under vacuum. As tissue is drawn into the opening of cutting member 130, the tissue is resected via the rotation and/or translation of cutting member 130 relative to shaft 120, thus enabling the resected tissue to be drawn proximally through cutting member 130, along with fluid and debris. The resected tissue, fluid, and debris are drawn proximally under vacuum through cutting member 130, through outflow port 150, and through outflow tubing (not shown) and, ultimately, to one or more collection canisters of a fluid management system (not shown).

Referring still to FIG. 1, access instrument 200 may be configured as a hysteroscope including an elongated tubular member 202 and a proximal body 240. Elongated tubular member 202 defines a working channel 204 configured to receive a working instrument therethrough, e.g., working instrument 100. Working channel 204 may also serve as a fluid inflow (or outflow) channel. Alternatively or additionally, a separate fluid inflow (or outflow) channel may be provided. Elongated tubular member 202 further includes optics 210 extending therethrough to enable visualization at the distal end of elongated tubular member 202.

Proximal body 240 includes a housing 242, a light post 244, a valve 246, and an arm 248. Light post 244 extends from housing 242 and is configured to connect to a light source, e.g., to illuminate a distal end of elongated tubular member 202 via one or more fiber optic strands (not shown) coupled to light post 244 and extending through elongated tubular member 202. Valve 246 is disposed in fluid communication with working channel 204 and is configured to enable the selective inflow and/or outflow of fluid to/from working channel 204. In configurations where multiple flow channels are provided, multiple valves may likewise be provided. Arm 248 is configured to connect to an imaging device, e.g., a camera, to capture images received via optics 210 and, thus, enable display of a video image of an internal surgical site as captured by optics 210.

Guide instrument 300 may be configured as an ultrasound device including a proximal body 310, a shaft 320 extending distally from proximal body 310, and an ultrasound sensor assembly 330 disposed at a distal end portion 324 of shaft 320. Guide instrument 300 further includes a longitudinal lumen 340 defined through proximal body 310 and shaft 320 and including an open proximal end 342 and an open distal end 344. Longitudinal lumen 340 may be coaxial with a longitudinal axis defined through shaft 320 or may be offset and/or angled relative thereto. Longitudinal lumen 340 is configured to permit passage of at least a portion of elongated tubular member 202 of access instrument 200 therethrough such that a distal portion of the elongated tubular member 202 extends through open distal end 344 of longitudinal lumen 340 and distally from shaft 320. Longitudinal lumen 340 may additionally or alternatively be configured to permit passage of other instrumentation, e.g., one or more working instruments, therethrough.

Proximal body 310 of guide instrument 300 may be configured as a handle including, for example, a pistol-style grip 312, although other handle configurations are also contemplated as are non-handle configurations, e.g., for mounting guide instrument 300 and/or attaching guide instrument 300 to a surgical robot arm. A cable 350 extends from proximal body 310 to connect ultrasound sensor assembly 330 to an ultrasound console (not shown), e.g., via wires (not shown) extending from ultrasound sensor assembly 330 through shaft 320, proximal body 310, and cable 350.

Shaft 320 of guide instrument 300 is configured for trans-vaginal insertion into position adjacent or in abutment with the cervix such that distal end 344 of longitudinal lumen 340 is oriented towards the cervical canal to enable passage of access instrument 200 (or other suitable surgical instrumentation) through longitudinal lumen 340, out distal end 344 thereof, through the cervical canal, and into the uterus. A body portion 322 of shaft 320 may define a cylindrical-shaped configuration and/or distal end portion 324 of shaft 320 may be tapered, curved, and/or otherwise atraumatically configured to facilitate atraumatic insertion.

Ultrasound sensor assembly 330 includes one or more ultrasound sensors 332, e.g., ultrasound transducers, to enable ultrasound imaging of tissue, e.g., the uterus. Each ultrasound sensor 332 is configured to emit ultrasound waves, e.g., high-frequency sound waves, and to receive echoed waves produced by the reflection of the ultrasound waves against the various tissue structures encountered. The echoed waves received by each ultrasound sensor 332 are output to an image processing unit (not shown), e.g., by way of wires extending through shaft 320, proximal body 310, and cable 350. In aspects, ultrasound sensor assembly 330 may be configured for 2D ultrasound imaging. In other aspects, ultrasound sensor assembly 330 includes a plurality of ultrasound sensors 332 forming an ultrasound sensor array that defines a portion of a circle, a portion of a polygon, a partially-polygonal, partially-arcuate configuration, or other suitable configuration to enable reconstruction of a 3D ultrasound image therefrom for 3D ultrasound imaging. In this manner, when activated, ultrasound sensor assembly 330 enables ultrasound imaging of tissue, e.g., the cervix, uterus, and/or surrounding tissue.

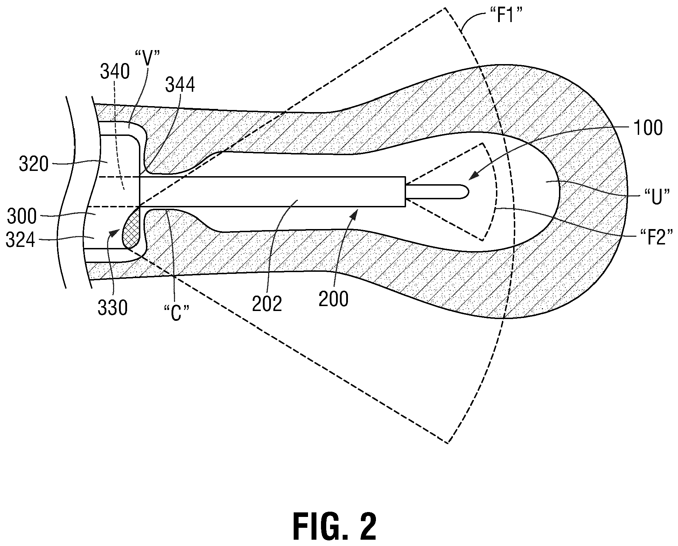

Turning to FIG. 2, system 10 is shown in use wherein guide instrument 300 is inserted trans-vaginally through the vaginal canal "V" such that distal end portion 324 of shaft 320 thereof is disposed adjacent to or in abutment with the cervix "C." In this position, more specifically, ultrasound sensor assembly 330 is positioned adjacent to or in abutment with the cervix "C" to enable ultrasound imaging of a field of view "F1" including the uterus "U" and surrounding tissue. Further, in this position, open distal end 344 of longitudinal lumen 340 is oriented in alignment with the cervical canal and elongated tubular member 202 extends therefrom, through the cervix "C" and into the uterus "U." In this manner, access instrument 200 may be utilized for visualization within the uterus "U," e.g., providing a field of view "F2," together with or separately from the ultrasound imaging. Access instrument 200 may also be used for the introduction of fluid into and/or the removal of fluid from the uterus "U" and/or for passage of a working instrument 100, e.g., a tissue resection device, ablation device, biopsy device, etc., therethrough and into the uterus "U" to perform one or more hysteroscopic tasks therein (e.g., within the uterus "U") or therethrough (e.g., within the uterine wall). The use of ultrasound imaging of the uterus "U" from the exterior thereof and/or visualization of the uterus "U" from within the uterine cavity provides increased visibility for performing various different hysteroscopic tasks without the need to swap out instruments supporting different imaging modalities and/or providing different imaging perspectives.

Referring generally to FIGS. 1 and 2, as noted above, longitudinal lumen 340 of guide instrument 300 is configured to permit passage of at least a portion of elongated tubular member 202 of access instrument 200 therethrough. More specifically, access instrument 200 is configured for telescopic insertion through longitudinal lumen 340 of guide instrument 300 such that rotation and axial translation of access instrument 200 relative to guide instrument 300 are permitted while transverse motion and tilting of access instrument 200 relative to guide instrument 300 are substantially constrained. Thus, access instrument 200, when inserted through longitudinal lumen 340 of guide instrument 300, is only permitted to rotate relative to guide instrument 300 or axially translate through guide instrument 300.

Similarly with respect to working instrument 100 and access instrument 200, working channel 204 of access instrument 200 is configured to permit passage of at least a portion of shaft 120 of working instrument 100 therethrough. More specifically, working instrument 100 is configured for telescopic insertion through working channel 204 of access instrument 200 such that rotation and axial translation of working instrument 100 relative to access instrument 200 are permitted while transverse motion and tilting of working instrument 100 relative to access instrument 200 are substantially constrained. Thus, working instrument 100, when inserted through working channel 204 of access instrument 200, is only permitted to rotate relative to access instrument 200 or axially translate through access instrument 200.

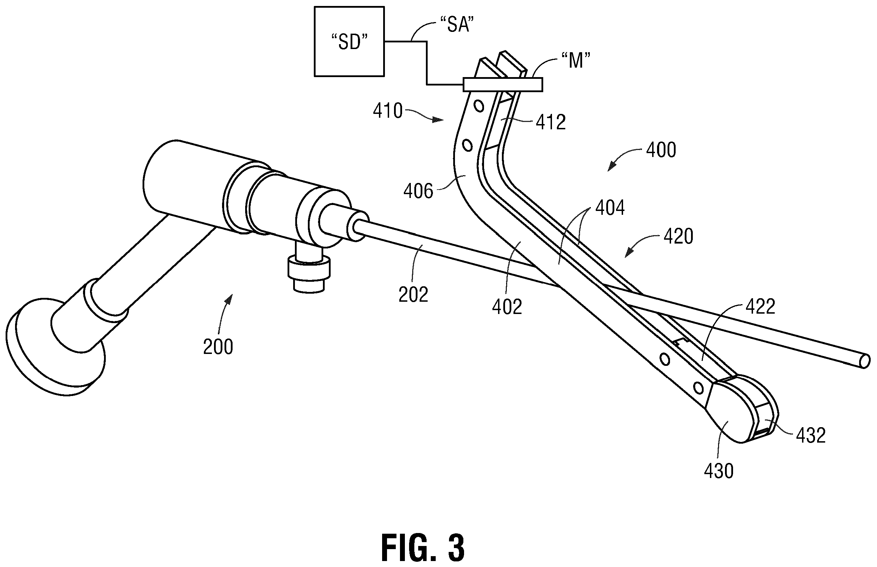

Turning to FIG. 3, other configurations of the access instrument and/or guide instrument to provide additional or alternative degrees of freedom of an instrument inserted therethrough and/or to constrain additional or alternative degrees of freedom of an instrument inserted therethrough are also contemplated. For example, FIG. 3 illustrates a guide instrument 400 provided in accordance with the present disclosure including access instrument 200 operably inserted therethrough.

Guide instrument 400 includes a frame 402 and an ultrasound sensor assembly 430. Frame 402 is formed from a pair of spaced-apart rails 404. Rails 404 may be configured as plates extending in substantially parallel orientation relative to one another, or in any other suitable manner. Rails 404 are bent at a bend 406 to define a more-proximal upright portion 410 and a more-distal longitudinal portion 420 disposed at an angle relative to one another, e.g., from about 60 degrees to about 120 degrees. A proximal spacer 412 is disposed between the rails 404 at upright portion 410 of frame 402 and is secured therebetween such that rails 404 are secured to one another and the spacing between rails 404 along upright portion 410 is maintained. A distal spacer 422 is disposed between the rails 404 at longitudinal portion 420 of frame 402 and is secured therebetween such that rails 404 are secured to one another and the spacing between rails 404 along longitudinal portion 420 is maintained.

The proximal end portion of upright portion 410 of frame 402 may include a handle disposed thereon to facilitate manual manipulation of guide instrument 400 and/or may be configured to be mounted on a support arm "SA" of a support device "SD," e.g., a robotic arm of a surgical system or a mounting arm of a mounting frame, thus obviating the need for a surgeon to manually manipulate guide instrument 400 and/or manually hold guide instrument 400 to retain the position thereof. Suitable mounting hardware "M" may be provided integrally with or attachable to the proximal end portion of upright portion 410 for releasably engaging support arm "SA."

Ultrasound sensor assembly 430 extends distally from the distal end of longitudinal portion 420 of frame 402 and is secured thereto. More specifically, ultrasound sensor assembly 430 may be formed with, attached to, or otherwise fixed relative to distal spacer 422 such that the engagement of distal spacer 422 between rails 404 also secures ultrasound sensor assembly 430 relative to frame 402, or may be secured relative to frame 402, directly or indirectly, in any other suitable manner. Ultrasound sensor assembly 430 includes one or more ultrasound sensors 432 and may be configured similar to and/or include any of the features of ultrasound sensor assembly 330 (FIGS. 1 and 2), as detailed above.

Bend 406 of frame 402 enables insertion of elongated tubular member 202 of access instrument 200 between rails 404 in substantially coaxial or parallel orientation relative to the longitudinal axis defined by longitudinal portion 420 of frame 402. Alternatively, elongated tubular member 202 of access instrument 200 may be inserted between rails 404 at an angle relative to the longitudinal axis. Regardless of the angle of insertion, with elongated tubular member 202 of access instrument 200 extending between rails 404, rails 404 serve to substantially inhibit transverse motion and tilting of elongated tubular member 202 in a first direction (laterally, from the orientation illustrated in FIG. 3) that is perpendicular to the longitudinal axis defined by longitudinal portion 420 of frame 402, while permitting transverse motion and tilting of elongated tubular member 202 in a second direction (vertically, from the orientation illustrated in FIG. 3) that is perpendicular to the longitudinal axis and the first direction. Frame 402 also permits rotation and longitudinal translation of elongated tubular member 202 relative to the longitudinal axis defined by longitudinal portion 420 of frame 402. Thus, access instrument 200, when inserted between rails 404 of guide instrument 400, is only permitted to rotate relative to guide instrument 400, longitudinally translate through guide instrument 400, and move and tilt transversely relative to guide instrument 400 in one direction perpendicular to the longitudinal axis of longitudinal portion 420 of frame 402.

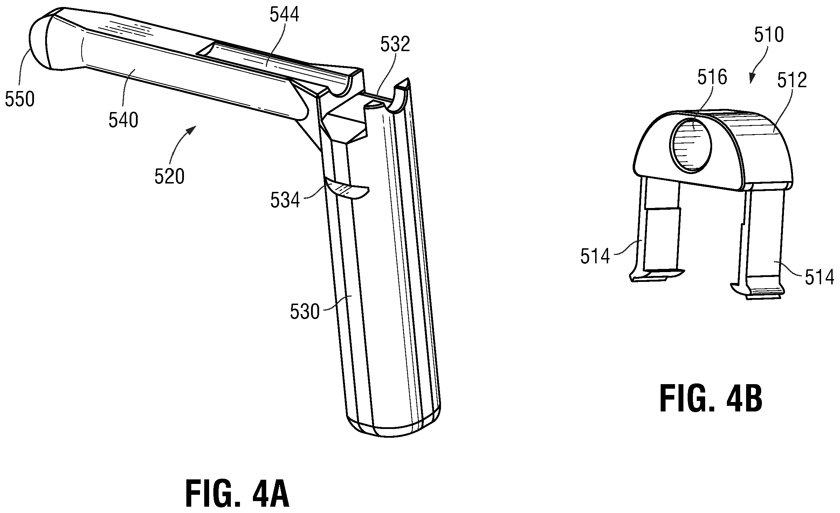

Referring to FIGS. 4A-4C, another guide instrument provided in accordance with the present disclosure is shown generally identified by reference numeral 500. Guide instrument 500 includes a guide piece 510 and a body 520. Guide piece 510 may be releasably engagable with body 520 (as shown) although integrated configurations are also contemplated. Body 520 includes a proximal handle 530 and a shaft 540 extending distally from proximal handle 530. Body 520 may be configured as an ultrasound device and, thus, may further include an ultrasound sensor assembly 550 disposed at a distal end of shaft 540. Ultrasound sensor assembly 550 may be configured similar to and/or include any of the features of ultrasound sensor assembly 330 (FIGS. 1 and 2), as detailed above.

Proximal handle 530 of body 520 is shown having a pistol-style grip, although other handle configurations are also contemplated as are non-handle configurations, e.g., for mounting body 520 on and/or attaching body 520 to a surgical robot arm. A cable (not shown) may extend from proximal handle 530 to connect ultrasound sensor assembly 550 to an ultrasound console (not shown), e.g., via wires (not shown) extending from ultrasound sensor assembly 550 through shaft 540, proximal handle 530, and the cable, although battery-powered configurations are also contemplated. Proximal handle 530 of body 520 further includes a saddle 532 disposed at an upper end thereof and an engagement notch 534 disposed on either side of saddle 532 at the upper end of proximal handle 530.

Shaft 540 of body 520 extends in a generally perpendicular orientation from the upper end of proximal handle 530, although other angles are also contemplated. Shaft 540 of body 520 defines a longitudinal recess 544 extending from a proximal end of shaft 540 distally along at least a portion of the length of shaft 540. Longitudinal recess 544 may define a semicylindrical configuration or any other suitable configuration and is configured to receive a portion of the instrument configured for coupling with guide instrument 500 to align and guide the instrument relative to body 520.

Guide piece 510 includes a head 512 and a pair of engagement tangs 514 extending from either side of head 512. Tangs 514 are configured to releasably engage engagement notches 534 of body 520, e.g., in snap-fit engagement, to secure guide piece 510 relative to body 520. Head 512 defines a guide aperture 516 that aligns with longitudinal recess 544 of body 520 when guide piece 510 is engaged with body 520. Guide instrument 500 is configured to telescopically receive, through guide aperture 516 and longitudinal recess 544, an instrument such that, similarly as detailed above with respect to guide instrument 300 (FIGS. 1 and 2), rotation and axial translation of the instrument relative to guide instrument 500 is permitted while transverse motion and tilting of the instrument relative to guide instrument 500 are substantially constrained. Thus, the instrument, when inserted through aperture 516 and longitudinal recess 544, is only permitted to rotate relative to guide instrument 500 or axially translate through guide instrument 500.

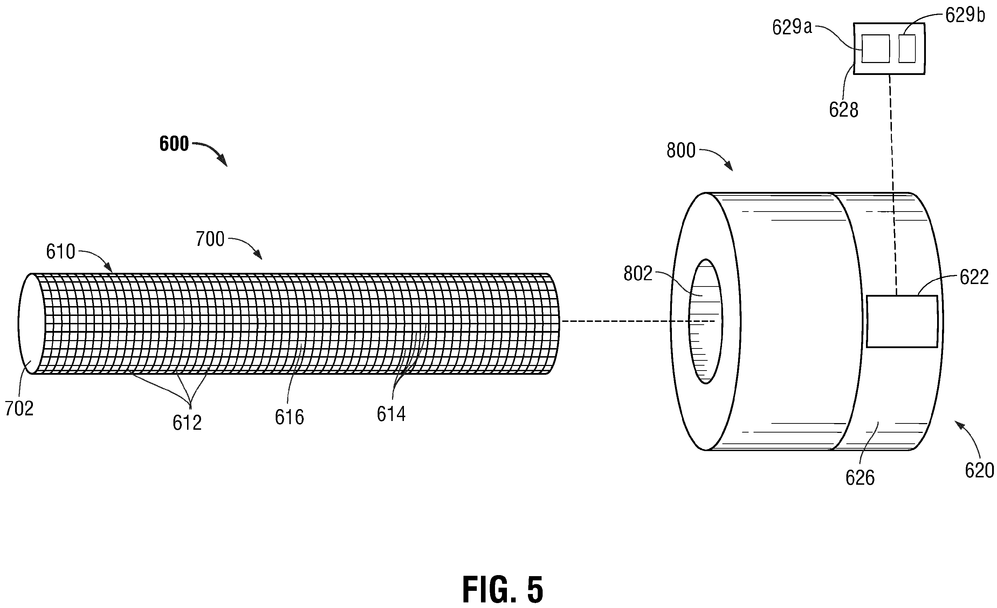

Turning to FIGS. 5-9, provided in accordance with the present disclosure are tracking devices, systems for tracking relative translational and rotational motion of one surgical instrument (e.g., one of instruments 100, 200, or any other suitable surgical instrument) relative to another surgical instrument (e.g., one of instruments 200, 300, 400, 500, or any other suitable surgical instrument).

With reference to FIG. 5, a tracking system 600 provided in accordance with the present disclosure includes a tracking pattern 610 and a tracking sensor assembly 620.

Tracking pattern 610 is disposed on a surgical instrument 700 and, more specifically, on a substantially cylindrical portion 702 of surgical instrument 700, which may represent, for example: a portion of shaft 120 of surgical instrument 100 (FIG. 1); a portion of elongated tubular member 202 of surgical instrument 200 (FIG. 1); etc. Tracking pattern 610 may be permanently disposed on surgical instrument 700, e.g., via providing tracking pattern 610 directly on surgical instrument 700, or may be removable from surgical instrument 700, e.g., tracking pattern 610 may be defined on a sleeve configured for positioning about surgical instrument 700, a sticker configured to adhere to surgical instrument 700, etc.

Tracking pattern 610 includes a plurality of longitudinal markers 612 longitudinally spaced at pre-determined (and, in aspects, constant) intervals and extending circumferentially about tracking pattern 610, and a plurality of rotational markers 614 circumferentially spaced at pre-determined (and, in aspects, constant) intervals and extending longitudinally along tracking pattern 610. As shown in FIG. 5, markers 612, 614 may be configured as lines forming a grid extending about the outer substantially cylindrical surface of portion 702 of surgical instrument 700, although other suitable patterns of markers 612, 614 and/or configurations (e.g., symbols, dots, etc.) of markers 612, 614 are also contemplated.

Markers 612, 614 may be painted, deposited, printed, etched, molded, sprayed, sputtered, or otherwise formed on surgical instrument 700 or a component (e.g., a sleeve or a sticker) configured to attach to surgical instrument 700. Tracking pattern 610 further includes a background 616 that contrasts with markers 612, 614, although in other aspects the underlying surgical instrument 700 and/or component functions as the contrast background 616.

Markers 612, 614 are configured to be detected via one or more sensors when markers 612, 614 move past the sensor(s) or otherwise intersect a sensing region associated with the sensor(s). More specifically, markers 612, 614 may be configured to be detected by optical sensors and, thus, are optically distinct, e.g., in color, reflectance, etc. from background 616, thus enabling the optical sensor to detect the presence of a marker 612, 614. In aspects, markers 612, 614 are also optically distinct from one another to enable an optical sensor to distinguish between markers 612, 614. In addition, longitudinal markers 612 may be optically distinguished from other longitudinal markers 612 and/or rotational markers 614 may be optically distinguished from other rotational markers 614.

In other aspects, markers 612, 614 may be configured to be detected by magnetic sensors and, thus, are electromagnetically distinct from background 616, e.g., wherein markers 612, 614 are magnetic and background 616 is not, vice versa, or where markers 612, 614 define a first electromagnetic property and background 616 defines a second, different electromagnetic property, thus enabling the magnetic sensors to detect the presence of a marker 612, 614. In such aspects, markers 612, 614 may also be electromagnetically distinct from one another. In addition, longitudinal markers 612 may be electromagnetically distinguished from other longitudinal markers 612 and/or rotational markers 614 may be electromagnetically distinguished from other rotational markers 614.

According to the invention, markers 612, 614 are configured to be detected by proximity sensors and, thus, are physically distinct from background 616, e.g., wherein markers 612, 614 protrude from or are recessed relative to background 616, thus enabling the proximity sensor to detect the presence of a marker 612, 614. According to the invention, markers 612, 614 are also physically distinct from one another, via protruding or being recessed more or less relative to one another or where one protrudes and the other is recessed. In addition, longitudinal markers 612 may be physically distinguished from other longitudinal markers 612 and/or rotational markers 614 may be physically distinguished from other rotational markers 614.

Other suitable configurations of markers 612, 614 relative to background 616 (and, in aspects, one another) to enable detection thereof via different sensors are also contemplated. Further, in aspects, markers 612 may be configured to be detected by a first type of sensor while markers 614 are configured to be detected by a second, different type of sensor.

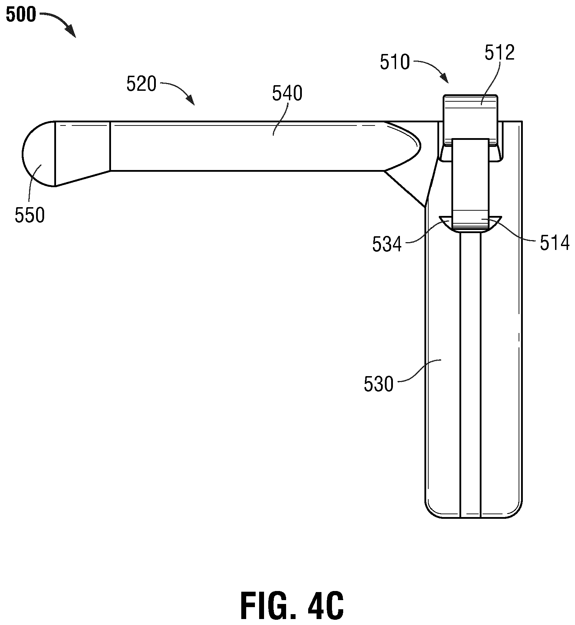

Continuing with reference to FIG. 5, sensor assembly 620 is associated with a surgical instrument 800 configured to operably receive cylindrical portion 702 of surgical instrument 700. More specifically, surgical instrument 800 includes a passage 802 (e.g., an opening, channel, aperture, lumen, etc.) configured to receive cylindrical portion 702 of surgical instrument 700, permitting rotation and translation of cylindrical portion 702 of surgical instrument 700, and constraining cylindrical portion 702 of surgical instrument 700 in at least one other degree of freedom. For example, surgical instrument 800 and passage 802 thereof may represent: working channel 204 of instrument 200 (FIG. 1); longitudinal lumen 340 of instrument 300 (FIG. 1); the space defined between rails 404 of instrument 400 (FIG. 3); or aperture 516 of instrument 500 (FIG. 4). Sensor assembly 620, more specifically, may be integrally formed with surgical instrument 800 or may be releasably engagable therewith. In either configuration, sensor assembly 620 may be disposed along passage 802 or at an end (adjacent or spaced-apart) of passage 802.

Sensor assembly 620 includes one or more sensors 622 disposed within a base 626 or integrated into surgical instrument 800. Further, processing electronics 628 may likewise be disposed within base 626 or integrated into surgical instrument 800, or may be remotely disposed (e.g., on or within a console) and electrically coupled to sensors 622 via a wired or wireless connection. Each sensor 622 or set of sensors 622 is configured to detect markers 612, 614 as markers 612, 614 move past the sensor 622 or otherwise intersect a sensing region associated with the sensor 622. Sensors 622, more specifically, are positioned to detect markers 612 as cylindrical portion 702 of surgical instrument 700 is longitudinally translated through and relative to passage 802 of surgical instrument 800 and to detect markers 614 as cylindrical portion 702 of surgical instrument 700 is rotated within and relative to passage 802 of surgical instrument 800. Sensors 622 may thus be directed inwardly towards passage 802, e.g., in configurations where sensor assembly 620 is disposed along passage 802, or may be directed inwardly towards an extension volume of passage 802, e.g., in configurations where sensor assembly 620 is disposed at an end of passage 802. In either configuration, the sensing regions associated with sensors 622 extend, e.g., transversely, through passage 802 (or a volume extension of passage 802), thereby enabling detection of markers 612, 614 as cylindrical portion 702 of surgical instrument 700 is moved relative to passage 802 of surgical instrument 800.

Depending upon the configuration of markers 612, 614, the one or more sensors 622 of sensor assembly 620 include a proximity sensor and may be optical sensors, magnetic sensors, combinations thereof, or any other suitable sensors or combinations of sensors to detect markers 612, 614.

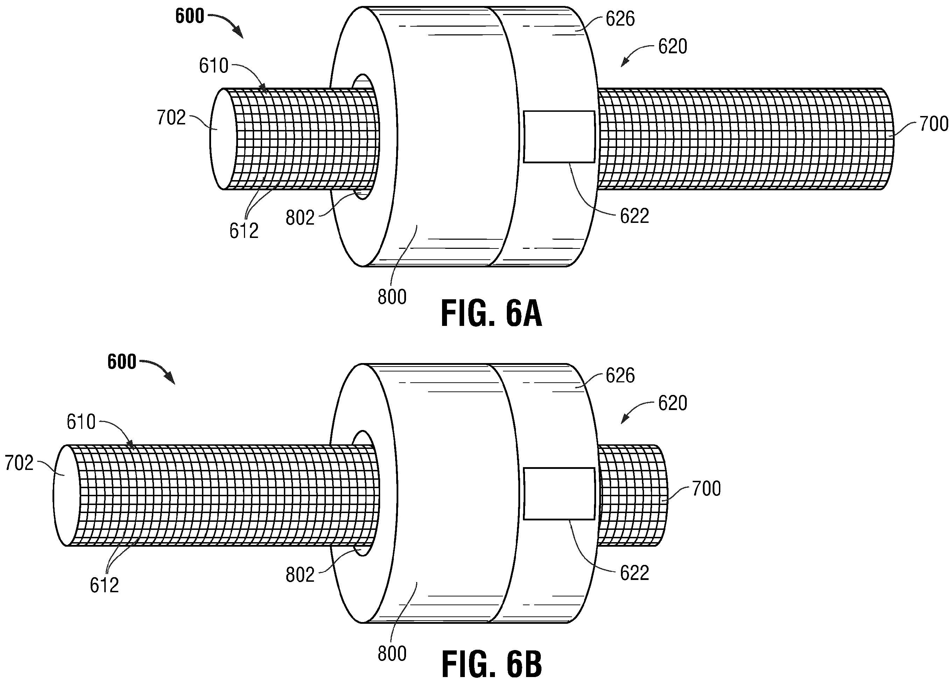

Turning now to FIGS. 6A and 6B, in use, as cylindrical portion 702 of surgical instrument 700 is translated longitudinally through passage 802 of surgical instrument 800, one or more of the sensors 622 of sensor assembly 620 detects the longitudinal markers 612 of pattern 610 moving past the sensor(s) 622. With additional reference to FIG. 5, the one or more sensors 622 are configured to communicate these detections (e.g., in the form of counts or other suitable signals) to processing electronics 628, which may include a processor 629a and memory 629b (e.g., a non-transitory computer-readable storage medium) storing instructions to be executed by processor 629a to process the detection signals received form sensor(s) 622. Processing electronics 628 may also include other suitable electronics such as, for example, an input/output (not explicitly shown) to enable communication with sensors 622 (via wired or wireless connection) and/or other devices, and/or, in aspects where processing electronics 628 are incorporated on or in base 626, a power source such as a battery (not explicitly shown). Processing electronics 628 are configured to count the number of markers 612 detected based upon the signal received from sensor(s) 622 and, given the known longitudinal spacing of the markers 612, determine the longitudinal distance cylindrical portion 702 of surgical instrument 700 has translated through passage 802. Likewise, by determining the rate at which the markers 612 are detected (e.g., where the signals provided by sensors 622 to processing electronics 628 are timestamped or include timestamp information), the speed of longitudinal translation of cylindrical portion 702 of surgical instrument 700 through passage 802 can also be readily determined by processing electronics 628. Markers 612 may include different proximal and distal edges (or other suitable differences) to further enable determination of a direction of translation of cylindrical portion 702 of surgical instrument 700 through passage 802. Additionally, or alternatively, as detailed below, the use of multiple sensors 622 enables directional tracking.

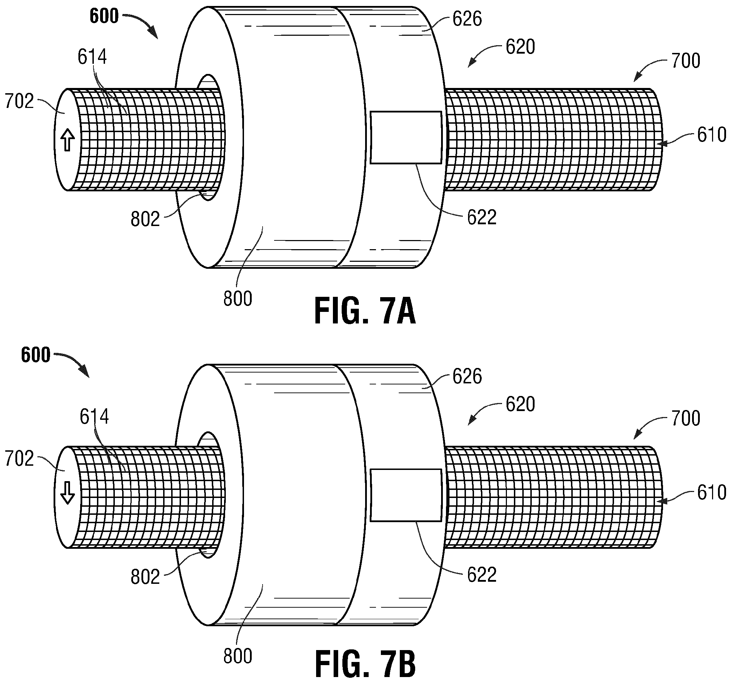

Referring to FIGS. 7A and 7B, in use, as cylindrical portion 702 of surgical instrument 700 is rotated within and relative to passage 802 of surgical instrument 800, one or more of the sensors 622 of sensor assembly 620 detects the rotational markers 614 of pattern 610 moving past the sensor(s) 622. By counting the number of markers 614 detected, and given the known angular spacing of the markers 614, the angular rotation of cylindrical portion 702 of surgical instrument 700 relative to passage 802 can be readily determined. Likewise, by detecting the rate at which the markers 614 are detected, the rotational speed of cylindrical portion 702 of surgical instrument 700 within passage 802 can also be readily determined. Markers 614 may include different clockwise-facing and counterclockwise-facing edges (or other suitable differences) to further enable determination of a direction of rotation of cylindrical portion 702 of surgical instrument 700 within passage 802. Additionally, or alternatively, as detailed below, the use of multiple sensors 622 enables directional tracking.

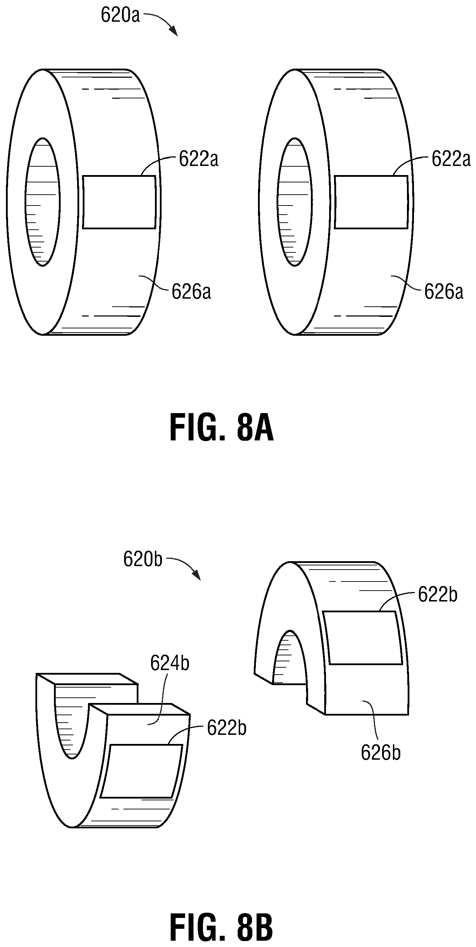

Turning to FIGS. 8A-8E, and with initial reference to FIG. 8A, in aspects, the sensor assembly 620a includes two bases 624a, 626a each including one or more sensors 622a. Bases 624a, 626a are longitudinally spaced-apart from one another such that the sensors 622a are likewise longitudinally spaced-apart from one another. Thus, in use, the direction of translation can be determined depending upon which sensor 622a detects a particular marker 612 or set or markers 612 first. Referring to FIG. 8B, similarly with respect to rotation, the sensor assembly 620b may include two bases 624b, 626b each including one or more sensors 622b. Bases 624b, 626b are circumferentially spaced-apart from one another such that the sensors 622b are likewise circumferentially spaced-apart from one another. Thus, in use, the direction of rotation can be determined depending upon which sensor 622b detects a particular marker 614 or set or markers 614 first.

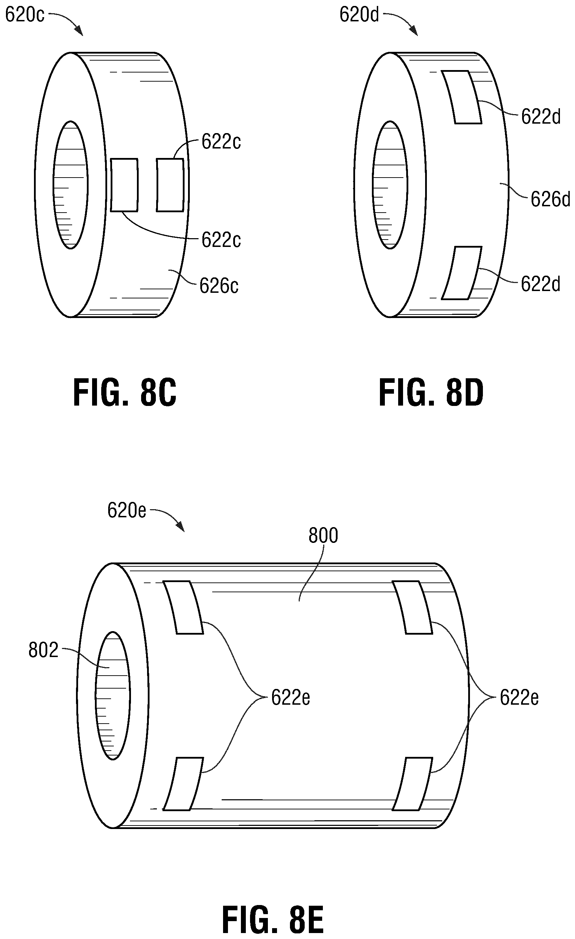

Referring to FIG. 8C, rather than two bases as with sensor assembly 620a (FIG. 8A), sensor assembly 620c may include longitudinally-spaced sensors 622c disposed within a single base 626c but may otherwise operate similar to sensor assembly 620a (FIG. 8A). Likewise with respect to FIG. 8D, rather than two bases as with sensor assembly 620b (FIG. 8B), sensor assembly 620d may include circumferentially-spaced sensors 622d disposed within a single base 626d but may otherwise operate similar to sensor assembly 620b (FIG. 8B). Combinations of the above-noted configurations in FIGS. 8A-8D are also contemplated.

Turning to FIG. 8E, rather than including a base, sensor assembly 620e may be integrated into a portion of surgical instrument 800. More specifically, sensors 622e may be disposed on or within surgical instrument 800 and operably positioned relative to passage 802 in any of the manners detailed above, combinations thereof, or in any other suitable manner to enable translational and/or rotational tracking of a surgical instrument inserted through passage 802, e.g., surgical instrument 700 (FIGS. 5-7B).

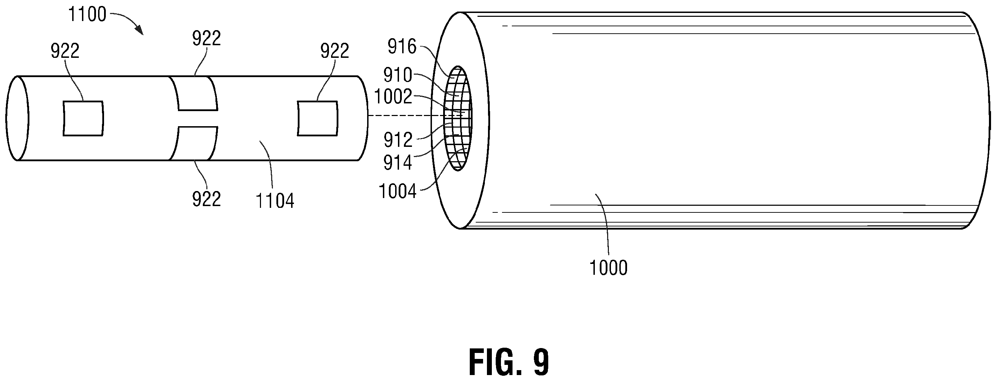

With reference to FIG. 9, in aspects, rather than the pattern being associated with the inner or guided instrument and the sensor assembly associated with the outer or guiding instrument, this configuration may be reversed. That is, a pattern 910 including markers 912, 914 (and, in aspects, a contrast background 916) including any of the aspects and features detailed hereinabove may be provided on an interior surface 1004 of a surgical instrument 1000 (e.g., a cylindrical interior surface 1004 of surgical instrument 1000) defining a passage 1002 at least partially through the surgical instrument 1000 while the one or more sensors 922 including any of the aspects and features detailed hereinabove are coupled to or integrated with a surgical instrument 1100 (e.g., a cylindrical portion 1104 of surgical instrument 1100) configured for insertion at least partially through the passage 1002 of the surgical instrument 1000.

Referring generally to FIGS. 5-9, in configurations where the surgical instrument to be tracked is configured to move transversely and/or tilt relative to the guide instrument, additional markings or configurations of the markings may be provided to enable tilt and/or transverse tracking.

Further, regardless of the direction and/or motion to be tracked, the sensor assemblies and sensors of the present disclosure may be configured for positioning at known locations relative to the guide instrument and/or any other suitable reference point and the markers may be located at known positions on the surgical instrument to be tracked. In this manner, the position and orientation of a surgical instrument to be tracked (or portion thereof, such as a distal end or end effector) may be determined relative to the guide instrument and/or reference point. In particular, with the guide instrument positioned at a particular anatomical location or located within a field of view, the position and orientation of the surgical instrument to be tracked (or portion thereof) may be determined relative to the anatomical location or position within the field of view such that, even if the surgical instrument (or portion thereof) to be tracked is not visible, the position thereof can be readily determined and visualized.

The determination of an amount of longitudinal translation and/or rotation of an instrument to be tracked relative to a guide instrument or a reference point, and/or the determination of a longitudinal and/or rotational position of the instrument to be tracked relative to the guide instrument or the reference point based on the amount of longitudinal translation and/or rotation, as in any of the above aspects, may be performed by suitable electronics (e.g., processing electronics 628 (FIG. 5) and/or other suitable electronics including one or more processors and associated memories storing instructions). The electronics (e.g., processing electronics 628 (FIG. 5)), in any of the above aspects, may be associated with or incorporated into: the sensor assembly; an instrument such as, for example, the instrument to be tracked, the guide instrument, or any other suitable instrument or combinations of instruments; a console; a (wired or wirelessly) connected computer such as, for example, a smartphone, tablet, desktop computer, laptop computer, server (a cloud-based server, a local server, or a remote server); combinations thereof; or in any other suitable manner. In aspects, the electronics (e.g., processing electronics 628 (FIG. 5)) are implemented across two or mode assemblies, instruments, and/or computers communicatively coupled to one another.

Further, as noted above, the determined amount of movement and/or position by the electronics (e.g., processing electronics 628 (FIG. 5)) may be output to different electronics or may be utilized by the same electronics to provide a suitable output such as, for example: a warning (visual, audible, tactile, combinations thereof, etc.) where the determined amount of movement exceeds a threshold and/or where the position exceeds a boundary, extends outside a field of view, is sufficiently approximated relative to another instrument or tissue of interest, etc.; a visual display of the determined amount of movement and/or position including a trajectory or path based on the determined movement and position; a feedback signal to a control device (such as an energy generator to control the application or cessation of energy, a motion control device such as a robot to move or stop movement of an instrument, or other suitable control device); and/or any other suitable output.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular aspects.

## Claims

1. A surgical system (600) for surgical instrument tracking, the surgical system comprising:
a first surgical instrument (700) including a pattern (610) having a plurality of first markers (612, 614) arranged for detection in response to longitudinal translation of the first surgical instrument and a plurality of second markers (612, 614) arranged for detection in response to rotation of the first surgical instrument;
a second surgical instrument (800) configured to guide the first surgical instrument such that the first surgical instrument is permitted to translate longitudinally and rotate relative to the second surgical instrument, and such that the first surgical instrument is constrained relative to the second surgical instrument in at least one other degree of freedom; and
a sensor assembly (620) operably coupled to the second surgical instrument, the sensor assembly including at least one proximity sensor (622) configured to:
detect at least one first marker of the plurality of first markers, thereby enabling determination of longitudinal translation of the first surgical instrument relative to the second surgical instrument; and
detect at least one second marker of the plurality of second markers, thereby enabling determination of rotation of the first surgical instrument relative to the second surgical instrument, **characterised in that**
said first plurality of markers and said second plurality of markers are physically distinct from one another by protruding or being recessed more or less relative to one another or where one protrudes and the other is recessed, and wherein said first plurality of markers and said second plurality of are physically distinct from a background (616).

2. The surgical system according to claim 1, wherein the second surgical instrument defines a passage (802) configured to receive at least a portion of the first surgical instrument, and wherein the first surgical instrument is configured to longitudinally translate and rotate within and relative to the passage of the second surgical instrument; preferably wherein a sensing region of each of the at least one sensors intersects the passage or an extension volume of the passage.

3. The surgical system according to claim 1 or claim 2, wherein the pattern is disposed on an exterior cylindrical surface of the first surgical instrument.

4. The surgical system according to any of claims 1 to 3, wherein the sensor assembly is integrated into the second surgical instrument.

5. The surgical system according to any of claims 1 to 3, wherein the sensor assembly is removably coupled to the second surgical instrument.

6. The surgical system according to any of claims 1 to 5, wherein the at least one sensor includes:
at least one first sensor configured to detect the at least one first marker of the plurality of first markers; and
at least one second sensor configured to detect the at least one second marker of the plurality of second markers.

7. The surgical system according to any of claims 1 to 5, wherein the at least one sensor includes:
at least two first sensors, each of the at least two first sensors configured to detect the at least one first marker of the plurality of first markers; and
at least two second sensors, each of the at least two second sensors configured to detect the at least one second marker of the plurality of second.

8. The surgical system according to claim 7, wherein at least one of:
the at least two first sensors enable determination of a direction of the longitudinal translation of the first surgical instrument relative to the second surgical instrument; or
the at least two second sensors enable determination of a direction of the rotation of the first surgical instrument relative to the second surgical instrument; and/or wherein the at least one sensor is an optical sensor and wherein the plurality of first markers are optically distinct from the plurality of second markers.

9. The surgical system of claim 1 wherein the first markers are longitudinally spaced relative to one another and the second markers are circumferentially spaced relative to one another.

10. The system according to claim 9 wherein the at least one sensor includes:
at least two first sensors, each of the at least two first sensors configured to detect the at least one first marker of the plurality of first markers, wherein the at least two first sensors are longitudinally spaced relative to one another, preferably wherein the at least two first sensors enable determination of a direction of the longitudinal translation of the first surgical instrument relative to the second surgical instrument.

11. The system according to claim 9, wherein the at least one sensor includes:
at least two sensors, each of the at least two sensors configured to detect the at least one second marker of the plurality of second markers, wherein the at least two sensors are circumferentially spaced relative to one another; preferably wherein the at least two sensors enable determination of a direction of the rotation of the first surgical instrument relative to the second surgical instrument.

12. The system according to claim 10, wherein the at least one sensor includes:
at least two second sensors, each of the at least two second sensors configured to detect the at least one second marker of the plurality of second markers, wherein the at least two second sensors are circumferentially spaced relative to one another; preferably wherein the at least two second sensors enable determination of a direction of the rotation of the first surgical instrument relative to the second surgical instrument.

## Patentansprüche

1. Chirurgisches System (600) zum Verfolgen chirurgischer Instrumente, das chirurgische System umfassend:
ein erstes chirurgisches Instrument (700), das ein Muster (610) einschließt, das eine Vielzahl von ersten Markierungen (612, 614), die für eine Erkennung als Reaktion auf eine Längsverschiebung des ersten chirurgischen Instruments angeordnet sind, und eine Vielzahl von zweiten Markierungen (612, 614) aufweist, die für die Erkennung als Reaktion auf eine Drehung des ersten chirurgischen Instruments angeordnet sind;
ein zweites chirurgisches Instrument (800), das konfiguriert ist, um das erste chirurgische Instrument derart zu führen, dass es dem ersten chirurgischen Instrument gestattet ist, sich in Längsrichtung zu verschieben und relativ zu dem zweiten chirurgischen Instrument zu drehen, und derart, dass das erste chirurgische Instrument relativ zu dem zweiten chirurgischen Instrument in mindestens einem anderen Freiheitsgrad eingeschränkt ist; und
eine Sensoranordnung (620), die mit dem zweiten chirurgischen Instrument wirkgekoppelt ist, wobei die Sensoranordnung mindestens einen Näherungssensor (622) einschließt, der konfiguriert ist zum:
Erkennen mindestens einer ersten Markierung der Vielzahl von ersten Markierungen, wodurch eine Bestimmung die Längsverschiebung des ersten chirurgischen Instruments relativ zu dem zweiten chirurgischen Instrument ermöglicht wird; und
Erkennen mindestens einer zweiten Markierung der Vielzahl von zweiten Markierungen, wodurch die Bestimmung der Drehung des ersten chirurgischen Instruments relativ zu dem zweiten chirurgischen Instrument ermöglicht wird, **dadurch gekennzeichnet, dass** die erste Vielzahl von Markierungen und die zweite Vielzahl von Markierungen voneinander physisch verschieden sind, indem sie mehr oder weniger relativ zueinander vorstehen oder vertieft sind, oder wo eine vorsteht und die andere vertieft ist, und wobei die erste Vielzahl von Markierungen und die zweite Vielzahl von Markierungen von einem Hintergrund (616) physisch verschieden sind.

2. Chirurgisches System nach Anspruch 1, wobei das zweite chirurgische Instrument einen Durchgang (802) definiert, der konfiguriert ist, um mindestens einen Abschnitt des ersten chirurgischen Instruments aufzunehmen, und wobei das erste chirurgische Instrument konfiguriert ist, um sich innerhalb des und relativ zu dem Durchgang des zweiten chirurgischen Instruments in Längsrichtung zu verschieben und zu drehen; vorzugsweise wobei ein Erfassungsbereich jedes des mindestens einen Sensors den Durchgang oder ein Erweiterungsvolumen des Durchgangs schneidet.

3. Chirurgisches System nach Anspruch 1 oder 2, wobei das Muster auf einer äußeren zylindrischen Oberfläche des ersten chirurgischen Instruments eingerichtet ist.

4. Chirurgisches System nach einem der Ansprüche 1 bis 3, wobei die Sensoranordnung in das zweite chirurgische Instrument integriert ist.

5. Chirurgisches System nach einem der Ansprüche 1 bis 3, wobei die Sensoranordnung an das zweite chirurgische Instrument lösbar gekoppelt ist.

6. Chirurgisches System nach einem der Ansprüche 1 bis 5, wobei der mindestens eine Sensor einschließt:
mindestens einen ersten Sensor, der konfiguriert ist, um die mindestens eine erste Markierung der Vielzahl von ersten Markierungen zu erkennen; und
mindestens einen zweiten Sensor, der konfiguriert ist, um die mindestens eine zweite Markierung der Vielzahl von zweiten Markierungen zu erkennen.

7. Chirurgisches System nach einem der Ansprüche 1 bis 5, wobei der mindestens eine Sensor einschließt:
mindestens zwei erste Sensoren, wobei jeder der mindestens zwei ersten Sensoren konfiguriert ist, um die mindestens eine erste Markierung der Vielzahl von ersten Markierungen zu erkennen; und
mindestens zwei zweite Sensoren, wobei jeder der mindestens zwei zweiten Sensoren konfiguriert ist, um die mindestens eine zweite Markierung der Vielzahl von zweiten zu erkennen.

8. Chirurgisches System nach Anspruch 7, wobei mindestens eines von:
die mindestens zwei ersten Sensoren die Bestimmung einer Richtung der Längsverschiebung des ersten chirurgischen Instruments relativ zu dem zweiten chirurgischen Instrument ermöglichen; oder
die mindestens zwei zweiten Sensoren die Bestimmung einer Richtung der Drehung des ersten chirurgischen Instruments relativ zu dem zweiten chirurgischen Instrument ermöglichen; und/oder wobei der mindestens eine Sensor ein optischer Sensor ist und wobei die Vielzahl von ersten Markierungen von der Vielzahl von zweiten Markierungen optisch verschieden ist.

9. Chirurgisches System nach Anspruch 1, wobei die ersten Markierungen in Längsrichtung relativ zueinander beabstandet sind und die zweiten Markierungen in Umfangsrichtung relativ zueinander beabstandet sind.

10. System nach Anspruch 9, wobei der mindestens eine Sensor einschließt:
mindestens zwei erste Sensoren, wobei jeder der mindestens zwei ersten Sensoren konfiguriert ist, um die mindestens eine erste Markierung der Vielzahl von ersten Markierungen zu erkennen, wobei die mindestens zwei ersten Sensoren in Längsrichtung relativ zueinander beabstandet sind, vorzugsweise wobei die mindestens zwei ersten Sensoren die Bestimmung einer Richtung der Längsverschiebung des ersten chirurgischen Instruments relativ zu dem zweiten chirurgischen Instrument ermöglichen.

11. System nach Anspruch 9, wobei der mindestens eine Sensor einschließt:
mindestens zwei Sensoren, wobei jeder der mindestens zwei Sensoren konfiguriert ist, um die mindestens eine zweite Markierung der Vielzahl von zweiten Markierungen zu erkennen, wobei die mindestens zwei Sensoren relativ zueinander in Umfangsrichtung beabstandet sind; vorzugsweise wobei die mindestens zwei Sensoren die Bestimmung einer Richtung der Drehung des ersten chirurgischen Instruments relativ zu dem zweiten chirurgischen Instrument ermöglichen.

12. System nach Anspruch 10, wobei der mindestens eine Sensor einschließt:
mindestens zwei zweite Sensoren, wobei jeder der mindestens zwei zweiten Sensoren konfiguriert ist, um die mindestens eine zweite Markierung der Vielzahl von zweiten Markierungen zu erkennen, wobei die mindestens zwei zweiten Sensoren relativ zueinander in Umfangsrichtung beabstandet sind; vorzugsweise wobei die mindestens zwei zweiten Sensoren die Bestimmung einer Richtung der Drehung des ersten chirurgischen Instruments relativ zu dem zweiten chirurgischen Instrument ermöglichen.

## Revendications

1. Système chirurgical (600) pour un suivi d'instruments chirurgicaux, le système chirurgical comprenant :
un premier instrument chirurgical (700) comportant un motif (610) ayant une pluralité de premiers marqueurs (612, 614) agencés pour une détection en réponse à la translation longitudinale du premier instrument chirurgical et une pluralité de seconds marqueurs (612, 614) agencés pour une détection en réponse à la rotation du premier instrument chirurgical ;
un second instrument chirurgical (800) conçu pour guider le premier instrument chirurgical de telle sorte que le premier instrument chirurgical soit autorisé à se translater longitudinalement et à tourner par rapport au second instrument chirurgical, et de telle sorte que le premier instrument chirurgical soit contraint par rapport au second instrument chirurgical dans au moins un autre degré de liberté ; et
un ensemble capteur (620) couplé de manière opérationnelle au second instrument chirurgical, l'ensemble capteur comportant au moins un capteur de proximité (622) configuré pour :
détecter au moins un premier marqueur de la pluralité de premiers marqueurs, permettant ainsi la détermination de la translation longitudinale du premier instrument chirurgical par rapport au second instrument chirurgical ; et
détecter au moins un second marqueur de la pluralité de seconds marqueurs, permettant ainsi la détermination de la rotation du premier instrument chirurgical par rapport au second instrument chirurgical, **caractérisé en ce que** ladite première pluralité de marqueurs et ladite seconde pluralité de marqueurs sont physiquement distinctes l'une de l'autre en étant plus ou moins en saillie ou en retrait l'une par rapport à l'autre ou lorsque l'une fait saillie et l'autre est en retrait, et dans lequel ladite première pluralité de marqueurs et ladite seconde pluralité sont physiquement distinctes d'un arrière-plan (616).

2. Système chirurgical selon la revendication 1, dans lequel le second instrument chirurgical définit un passage (802) conçu pour recevoir au moins une partie du premier instrument chirurgical, et dans lequel le premier instrument chirurgical est conçu pour se translater longitudinalement et tourner à l'intérieur du passage du second instrument chirurgical et par rapport à celui-ci ; de préférence dans lequel une région de détection de chacun de l'au moins un capteur coupe le passage ou un volume d'extension du passage.

3. Système chirurgical selon la revendication 1 ou la revendication 2, dans lequel le motif est disposé sur une surface cylindrique extérieure du premier instrument chirurgical.

4. Système chirurgical selon l'une quelconque des revendications 1 à 3, dans lequel l'ensemble capteur est intégré au second instrument chirurgical.

5. Système chirurgical selon l'une quelconque des revendications 1 à 3, dans lequel l'ensemble capteur est accouplé de manière amovible au second instrument chirurgical.

6. Système chirurgical selon l'une quelconque des revendications 1 à 5, dans lequel l'au moins un capteur comporte :
au moins un premier capteur configuré pour détecter l'au moins un premier marqueur de la pluralité de premiers marqueurs ; et
au moins un second capteur configuré pour détecter l'au moins un second marqueur de la pluralité de seconds marqueurs.

7. Système chirurgical selon l'une quelconque des revendications 1 à 5, dans lequel l'au moins un capteur comporte :
au moins deux premiers capteurs, chacun des au moins deux premiers capteurs étant configuré pour détecter l'au moins un premier marqueur de la pluralité de premiers marqueurs ; et
au moins deux seconds capteurs, chacun des au moins deux seconds capteurs étant configuré pour détecter l'au moins un second marqueur de la pluralité de seconds.

8. Système chirurgical selon la revendication 7, dans lequel au moins l'un parmi :
les au moins deux premiers capteurs permettent la détermination d'un sens de la translation longitudinale du premier instrument chirurgical par rapport au second instrument chirurgical ; ou
les au moins deux seconds capteurs permettent la détermination d'un sens de la rotation du premier instrument chirurgical par rapport au second instrument chirurgical ; et/ou dans lequel l'au moins un capteur est un capteur optique et dans lequel la pluralité de premiers marqueurs est optiquement distincte de la pluralité de seconds marqueurs.

9. Système chirurgical selon la revendication 1, dans lequel les premiers marqueurs sont espacés longitudinalement les uns par rapport aux autres et les seconds marqueurs sont espacés circonférentiellement les uns par rapport aux autres.

10. Système chirurgical selon la revendication 9, dans lequel l'au moins un capteur comporte :
au moins deux premiers capteurs, chacun des au moins deux premiers capteurs étant configuré pour détecter l'au moins un premier marqueur de la pluralité de premiers marqueurs, dans lequel les au moins deux premiers capteurs sont espacés longitudinalement les uns par rapport aux autres, de préférence dans lequel les au moins deux premiers capteurs permettent la détermination d'un sens de la translation longitudinale du premier instrument chirurgical par rapport au second instrument chirurgical.

11. Système selon la revendication 9, dans lequel l'au moins un capteur comporte :
au moins deux capteurs, chacun des au moins deux capteurs étant configuré pour détecter l'au moins un second marqueur de la pluralité de seconds marqueurs, dans lequel les au moins deux capteurs sont espacés circonférentiellement les uns par rapport aux autres ; de préférence dans lequel les au moins deux capteurs permettent la détermination d'un sens de la rotation du premier instrument chirurgical par rapport au second instrument chirurgical.

12. Système selon la revendication 10, dans lequel l'au moins un capteur comporte :
au moins deux seconds capteurs, chacun des au moins deux seconds capteurs étant configuré pour détecter l'au moins un second marqueur de la pluralité de seconds marqueurs, dans lequel les au moins deux seconds capteurs sont espacés circonférentiellement les uns par rapport aux autres ; de préférence dans lequel les au moins deux seconds capteurs permettent la détermination d'un sens de la rotation du premier instrument chirurgical par rapport au second instrument chirurgical.
